# EUROPEAN PATENT SPECIFICATION

(11) **EP 1 390 099 B1**
(45) Date of publication and mention of the grant of the patent: **19.03.2008**
(21) Application number: 01930784.2
(22) Date of filing: 27.04.2001
(51) Int. Cl.: A61N 1/30, A61N 1/32

(54) **SYSTEM FOR ELECTROKINETIC DELIVERY OF A SUBSTANCE**
SYSTEM FÜR DIE ELEKTROKINETISCHE FREISETZUNG EINER SUBSTANZ
SYSTEME DESTINE A L'ADMINISTRATION ELECTROCINETIQUE D'UNE SUBSTANCE

(43) Date of publication of application: 25.02.2004
(73) Proprietor: Transport Pharmaceuticals, Inc., Framingham, MA 01701 (US)
(72) Inventor: CHANG, Kuo-Wei, Lexington, MA 02421 (US)
(74) Representative: Moir, Michael Christopher
(86) International application number: PCT/US2001/013431
(87) International publication number: WO 2002/087691

(56) References cited:
- GB-A- 1 485 170
- US-A- 5 019 034
- US-A- 5 042 975
- US-A- 5 135 478
- US-A- 5 551 953
- US-A- 5 676 648
- US-A- 5 954 684
- US-A- 5 961 482

## Description

### BACKGROUND OF THE INVENTION

The present invention generally relates to the electrokinetic delivery of a substance (for example, a medicament) into tissue and, more particularly, to a system for such delivery that satisfies certain risk criteria for medical equipment that maintains direct electrical contact with human skin. The majority of applications using the present invention are for applying medicaments to treatment sites and therefore the term medicament is used in lieu of the term substance throughout this description.

One type of electrokinetic delivery mechanism is iontophoresis. Iontophoresis is the transfer of ionic agents into tissue by means of electricity. The active component of the medicament, either directly ionizable or attached to a carrier ion and either positively or negatively charged, is driven into the tissue by a properly biased electrode through barriers such as animal (including human) skin, cell and mucosal membranes and other barrier surfaces. Iontophoresis has been used to deliver, among other things, morphine HCL for postoperative pain relief, topical anesthetics (such as lidocaine) for transdermal anesthetization, anti-viral agents for herpes infection, and anti-fungal medicines for athlete's foot. The use of iontophoretic transdermal or transmucocutaneous delivery techniques obviates the need for hypodermic injection for many medicaments, thereby eliminating the concomitant problems of trauma, pain and risk of infection to the patient. Such delivery techniques may also be utilized for controlled or localized treatment especially when a substantial systemic involvement of the medicament is unwanted or harmful.

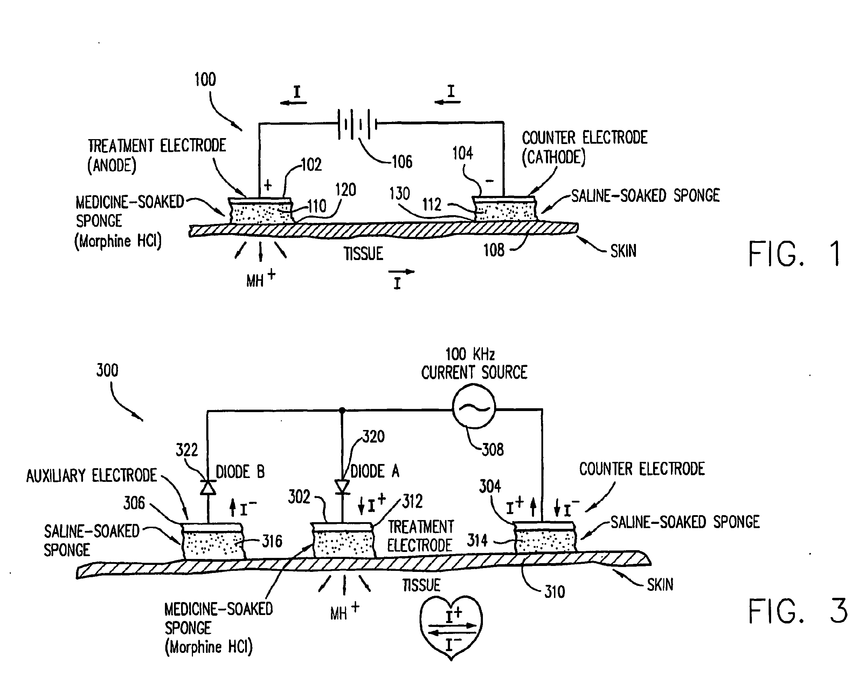

Regardless of the charge of the medicament to be administered, conventional iontophoretic delivery devices typically employ two electrodes (an anode and a cathode). In conjunction with the patient's skin or mucosa, the first (applicator or treatment) electrode is positioned at a treatment site on the skin or mucosa, and the second (counter) electrode is affixed to a second site on the skin or mucosa. These electrodes form a current path that enhances the rate of penetration of the medicament into the treatment site adjacent to the applicator electrode. A conventional iontophoretic delivery system 100 is shown in Fig. 1. System 100 includes a treatment electrode (anode) 102 and a counter electrode (cathode) 104 connected to a DC power supply 106. Electrodes 102 and 104 are in electrical contact with the skin or mucosa via conductive layers 110 and 112, respectively. Such layers may be part of a single medicament-carrying substrate. The medicament-carrying substrate is generally disposable and non-reusable and may be releasably adherable to the patient's treatment site and/or to electrodes 102 and 104. Conductive layers 110 and 112 are shown in Fig. 1 as comprising a medicine-soaked sponge (e.g., a morphine HCL-soaked sponge) and a saline-soaked sponge, respectively. In use, iontophoretic device 100 is turned on (e.g., by a switch, not shown) and a current flows from treatment electrode 102, through conductive layer 110 and skin plus underlying tissue 108, to counter electrode 104, thereby driving medicament through the treatment site into the skin and underlying tissue.

Although use of alternating current has been reported (see, e.g., U.S. Patent No. 5,224,927), direct current is generally preferred in iontophoresis. As set forth in the '927 patent, at AC frequencies higher than approximately 10 Hz, no substantial effective drug delivery takes place. Medicament and other ions merely move to and fro, lacking any net unidirectional movement. For DC iontophoresis, the amount of current used varies from 0.2 to 1 milliampere, which exceeds the risk-current limit of 10 microamperes for medical equipment that maintains direct electrical contact with the patient. There exists, therefore, a potential hazard associated with ventricular fibrillation and cardiac arrest if the current generated during iontophoresis accidentally passes through the patient's heart. In iontophoresis, the rate of drug delivery increases with current. For this reason, higher current is, in principle, always favored because treatment time is proportionally reduced. However, for current exceeding 0.5 to 1 milliampere, the patient will feel an uncomfortable burning sensation. Even at the 0.5 to 1 milliampere range, when the treatment area is relatively small, the resulting high current density can cause severe pain and actual burning and destruction of the skin tissue.

In any case, to remain effective, existing iontophoresis devices may use treatment currents exceeding the established risk-current limit. In order to reduce the ventricular fibrillation risk, some devices limit the separation distance between the treatment and the counter electrode so that the heart is not directly in the current path and is therefore less likely to be included within the fringe electric fields created by the electrodes. However, because electric current always flows through a path of least resistance, the electrode separation distance needs to be large enough so that current is not shortcircuited or concentrated between proximal edges of the electrodes (i.e., between edges 120 and 130 in Fig. 1), so that the current distribution under the treatment electrode is relatively uniform for effective drug delivery, and so that there are no hot-spots or areas of high current density to cause discomfort and pain. Such an approach for cardiac risk avoidance can be further compromised in situations where the patient can touch by fingers or hand the skin area near the electrodes such that the treatment current is diverted and passes through the heart via the arm. Some electrophoresis devices use a large separation distance to achieve a uniform current distribution but place the counter electrode in a less accessible location such as the back or the rear shoulder of the patient.

An effective method for self-administration of a medicament into an individual's skin is disclosed in U.S. Patent No. 5,676,648 and uses a small cylindrical probe in which the treatment applicator electrode is located at the distal end of a counter electrode consisting of a circumferential tactile metal band which provides electrical connection to the individual's finger and hand. The individual's body completes a long electrical circuit path (through the arm and torso), and thus a uniform current distribution and effective medicament delivery is assured.

U.S. 5,954,684 (Flower et al.) discloses an iontophoretic drug delivery system and method in which a DC power supply is connected to at least two electrodes in electrical contact with respective first and second reservoirs. The two reservoirs each contain an active agent to be delivered to an applied area of a patient. A timer is supplied to determine the time that electrical current flows to each electrode for delivering active agent to the patient from the respective reservoir.

### SUMMARY OF THE INVENTION

It is desirable to use a system for electrokinetic delivery of medicament into tissue that reduces hazards associated with introducing electrical currents into the human or animal body.

In accordance with the present invention, there is provided a system for delivering a substance into a body at a substance-delivery site, the system including: an alternating current source; a plurality of electrodes; and, circuitry connected between the alternating current source and the electrodes for supplying current to the electrodes when the electrodes are in electrical contact with the body, the circuitry comprising means for maintaining a unidirectional current flow at the substance-delivery site for delivering the substance into the body, and for maintaining a bi-directional current flow through the body.

As an example of an electrokinetic delivery mechanism, iontopheresis carried out as described above satisfies the established risk-current limit requirements and eliminates the hazard of ventricular fibrillation. In addition, unidirectional iontopheresis like that of the DC approach can be obtained.

The accompanying drawings, which are incorporated in and constitute a part of the specification, illustrate various embodiments of the present invention and, together with the general description given above and the detailed description provided below, serve to explain the principles of the invention.

### BRIEF DESCRIPTION OF THE DRAWINGS

Fig. 1 shows a conventional iontophoretic delivery system 100.
Fig. 2 is a graph of risk current (RMS) in microamperes versus frequency showing the risk current limits based on fibrillatory thresholds.
Fig. 3 shows an iontophoretic delivery system 300 in accordance with an embodiment of the present invention.
Fig. 4 shows a block diagram of electrical circuit elements of an embodiment of the present invention.
Fig. 5 shows a hand-held device with internal layout of electric and electronic elements.

### DETAILED DESCRIPTION OF THE INVENTION

The present invention is described in the context of exemplary embodiments. However, the scope of the invention is not limited to the particular examples and embodiments described in this specification. Rather the specification merely reflects certain embodiments and serves to illustrate the principles and characteristics of the present invention. Those skilled in the art will recognize that various modifications and refinements may be made without departing from the scope of the invention.

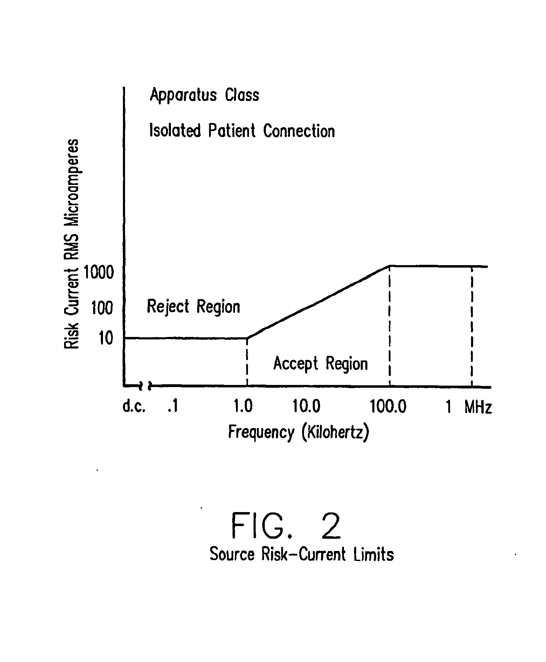

A system for the safe application of an electrokinetic delivery system, such as iontopheresis, is described with reference to Figs. 2 through 5. The system is based on the use of a high frequency rectified current in conjunction with three electrodes, referred to herein as a treatment electrode, a counter electrode and an auxiliary electrode. For purposes of illustration, transdermal delivery of morphine is used as an example. It will of course be appreciated that the system described herein is usable in connection with substances generally including natural or homeopathic products that may be outside the definition of medicaments as well as medicaments (e.g., lidocaine for transdermal anesthetization, anti-viral agents for herpes infections, and anti-fungal medicine for athlete's foot) and in connection with barriers other than skin (e.g., cell membranes, mucosal membranes, etc.). By medicament is meant any chemical or biologic that may be used on or administered to humans or animals as an aid in the diagnosis, treatments or prevention of disease or other abnormal or cosmetic condition or for the relief of pain or to control or improve any physiologic or pathologic condition.

As described above, iontophoresis involves the use a current to deliver a substance to tissue. In conventional systems, there is a potential hazard associated with ventricular fibrillation and cardiac arrest if the current generated during iontophoresis accidentally passes through the patient's heart. The standard current threshold for ventricular fibrillation risk increases with frequency. Fig. 2 is a graph of risk current (RMS) in microamperes versus frequency showing the risk current limits based on fibrillatory thresholds. For direct current (DC), the limit is 10 microamperes. For frequencies from 1 kilohertz to 100 kilohertz, the risk current limit varies from 10 microamperes to 1 milliampere. For frequencies above 100 kilohertz, but below 1 megahertz, the risk current limit remains at 1 milliampere. See, for example, AAMI (Association for the Advancement of Medical Instrumentation) Standard, "Safe Current Limits Standard."

Fig. 3 shows an iontophoretic delivery system 300 in accordance with an embodiment of the present invention. System 300 includes a treatment electrode 302, a counter electrode 304 and an auxiliary electrode 306 connected to a 100 kilohertz alternating current source 308. Electrodes 302, 304 and 306 are in electrical contact with the patient's skin via conductive layers 312, 314 and 316, respectively. Such layers may, for example, be part of a medicament-carrying substrate or pad. The medicament-carrying substrates or pads are generally disposable and non-reusable and may be releasably adherable to the patient's skin and/or to electrodes 302, 304 and 306. Conductive layer 312 is shown in Fig. 3 as comprising a medicament-soaked sponge or other porous open cellular material, such as cotton, and conductive layers 314 and 316 are shown in Fig. 3 as each comprising a saline-soaked sponge or other such material.

For example, conductive layer 312 may be of a mesh-like construction having vertical cells dimensioned to accommodate a viscous fluid within the confines of the cellular structures. The viscous fluid contained within the plurality of cells includes a medicament that is in a form suitable for transport under the influence of an electric current. Conductive layers 314 and 316 may be mesh-like tactile conductive portions that contain an electrically conductive gel or fluid therewithin. Each of the conductive layers has a lower skin-facing surface and an upper electrode-facing surface. The cells form apertures between the lower skin-facing surface and the upper electrode-facing surface. The device-facing surfaces of the electrodes may further include an adhesive layer applied thereto for suitably releasably adhering the electrodes to the iontophoresis device.

Auxiliary electrode 306 is located lateral to, behind or near treatment electrode 302. Auxiliary electrode 306 and treatment electrode 302 can be in close proximity to each other and the area of auxiliary electrode 306 can be very small compared to the area of treatment electrode 302. These features permit design of a compact hand-held unit in spite of the addition of an auxiliary electrode. In one particular implementation, the total area can be reduced to a minimum by placing auxiliary electrode 306, in the form of a metal mesh, in front of treatment electrode 302. The open mesh allows free passage of medicament and ions to and from treatment electrode 302. Of course, auxiliary electrode 306 may be positioned elsewhere and the present invention is not limited in this respect.

Treatment electrode 302 is connected to AC source 308 via a current path including a first rectifying element 320 for passing current flowing from AC source 308 to skin (and tissue) 310 and blocking current flowing from skin (and tissue) 310 to AC source 308. In the illustrative Fig. 3 embodiment, first rectifying element 320 is a diode having its anode connected to AC source 308 and its cathode connected to treatment electrode 302. Auxiliary electrode 306 is connected to AC source 308 via a current path including a second rectifying element 322 for passing current flowing from skin (and tissue) 310 to AC source 308 and blocking current flowing from AC source 308 to skin (and tissue) 310. In the illustrative Fig. 3 embodiment, second rectifying element 322 is a diode having its anode connected to auxiliary electrode 306 and its cathode connected to AC source 308. Counter electrode 304 is connected to AC source 308 via a bi-directional current path over which current can flow from AC source 308 to skin (and tissue) 310 and from skin (and tissue) 310 to AC source 308.

In use, treatment electrode 302, counter electrode 304, auxiliary electrode 306 are placed in electrical contact with skin 310 via conductive layers 312, 314 and 316, respectively. Conductive layers 312, 314 and 316 may be releasably attached to the electrodes and/or to skin 310 using, for example, a releasable adhesive. Iontophoretic system 300 is then turned on using, for example, a switch (not shown in Fig. 3). During the positive cycle portions of AC source 308, a component current I⁺ flows from treatment electrode 302 to the patient's skin and tissue and from the patient's skin and tissue to counter electrode 304. In this way, for example, morphine HCL ions (MH+) are delivered to the tissue covered by the patient's skin. During the negative cycle portions of AC source 308, a component current I⁻ flows from counter electrode 304 to the patient's skin and tissue and from the patient's skin and tissue to auxiliary electrode 306.

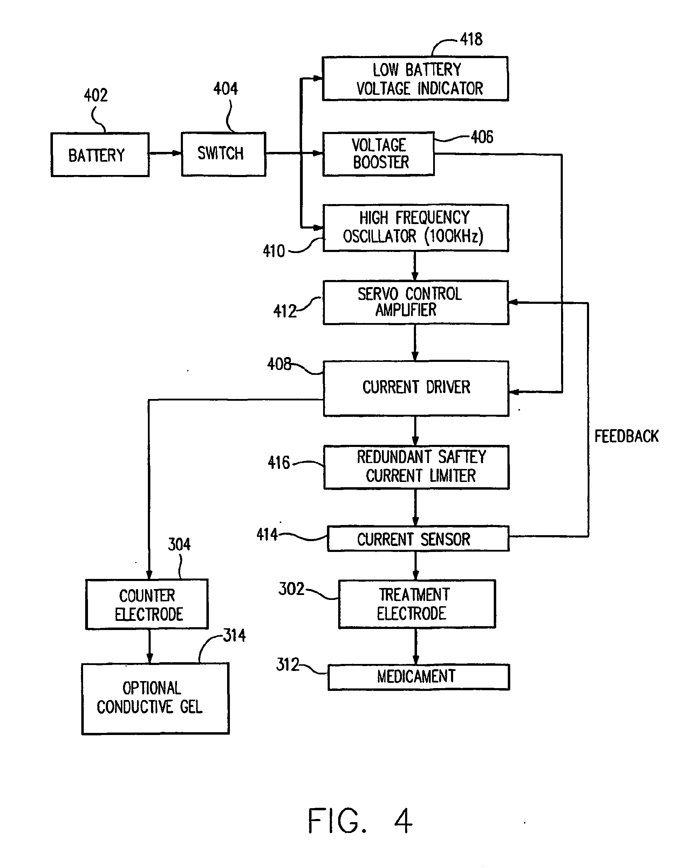

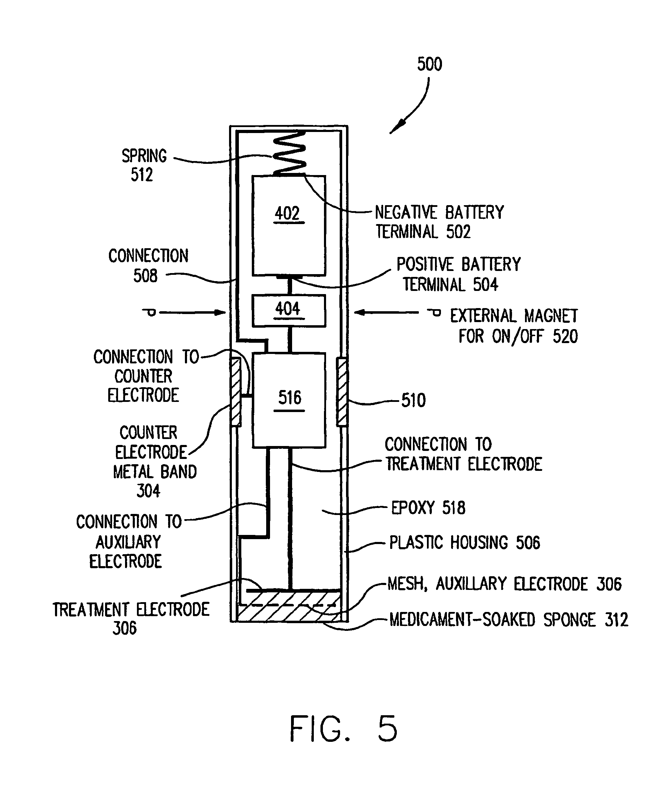

Fig. 4 is a block diagram showing electronic circuit design elements used in an illustrative implementation of a high frequency unidirectional iontophoretic medicator in accordance with an embodiment of the present invention. In this example, power source 402 is a battery comprising one or more AAA-sized primary cells connected either in series or in parallel. Counter electrode 304 is connected to an output of current driver 408 (see Figs. 4 and 5). An optional conductive layer 314 (such as a conductive gel or a saline-soaked sponge) is used to facilitate current flow to and from the patient's skin. An internal mechanical or electronic switch 404, activated externally by a magnet or magnetic material 520 (see Fig. 5), controls the on and off status of the device. A voltage booster circuit 406 converts the low battery voltage (e.g.,1.5 to 3 VDC) to a high voltage around 30 VDC. The high voltage or high potential is preferred to allow a current driver 408 to overcome any tissue resistance. An oscillator circuit 410 generates a square-wave or sinusoidal AC signal with the selected operating frequency (e.g., 100 kilohertz). A servo-controlled amplifier 412, in synchronization with the oscillator signal, controls the current magnitude based on current feedback signals from a current sensor 414. A current driver stage 408 controls the bias voltage and maintains the desired current to treatment electrode 302. A redundant current limiter 416 is used to provide a safe upper limit for the treatment current. A low battery voltage indicator 418 (e.g., an LED) signals when the battery capacity is low.

Fig. 5 illustrates a hand-held device 500 into which the various circuit elements of Fig. 4 may be incorporated. Of course, it will be apparent that the circuit elements of Fig. 4 may be incorporated in a wide variety of devices and the device of Fig. 5 is provided by way of illustration, not limitation. The hand-held device shown in Fig. 5 is configured along the lines of the hand-held devices shown in U.S. Patent Nos. 5,676,648, 5,879,323 and 5,908,401,.
For ease of illustration, not all of the elements shown in Fig. 4 are shown in Fig. 5. Housing 506 of the handheld device is preferably formed of plastic and is shaped to comfortably fit within a user's hand. Medicament-soaked sponge 312 is in electrical contact with treatment electrode 306. A mesh or gridded auxiliary electrode 306 is, as an example, interposed between treatment electrode 306 and the patient. Negative battery terminal 502 is connected via an electrical connection 508 to an electronic package 516, and an output of a current drive circuit within electronic package 516 connects to counter electrode 304 provided as a metal band 510 circumferentially (either continuously or discontinuously) formed around housing 506. For the self-administration of medicament, a user touches counter electrode 304 with his/her skin (e.g., fingers). Electrical connection 508 includes a spring portion 512 for holding power source (battery) 402 in place. Positive battery terminal 504 is connected to switch 404 (e.g, a mechanical reed switch or an electronic switch activated by an external magnet denoted at 520). Element 516 in Fig. 5 designates an electronic package at least containing oscillator 410, amplifier 412, current driver 408, redundant safety current limiter 416, current sensor 414 and rectifying elements 320 and 322. Some or all of the components within housing 506 may be contained in epoxy 518.

As described above, in cases in which iontophoresis treatments are administered by a patient without the supervision of medical professionals (e.g., at home), current may accidentally pass through the patient's heart. With conventional equipment, the portion of the current directly traversing the patient's heart could reach a level resulting in ventricular fibrillation. In accordance with the above-described embodiment of the present invention, the frequency of the electrical driving circuit is increased from 0 (DC) to 100 kilohertz. As can be seen with reference to Fig. 2, in this case, the current can be safely increased up to 1 milliampere (RMS). This results in effective delivery of the medicament to the patient. Thus, the use of rectified highfrequency iontopheresis as described above satisfies the established risk-current limit requirements and eliminates the hazard of ventricular fibrillation. In addition, the goal of unidirectional iontophoresis like that of the DC approach can be obtained. Therefore, although the AC current is rectified at the treatment site to obtain DC-like, unidirectional iontophoresis, any current passing through the heart remains strictly bi-directional and alternating with a frequency high enough to meet the risk current requirement.

In rare cases in which AC iontopheresis is applicable, the hazard associated with ventricular fibrillation can also be eliminated by using a high frequency current source around 100 kilohertz. In this special case, rectifying elements and auxiliary electrode 102 are not required because AC iontophoresis is desired. The same circuit design used for unidirectional AC electrophoresis (Fig. 4) is directly applicable.

While the invention has been described in connection with what is presently considered to be the most practical and preferred embodiment, it is to be understood that the invention is not to be limited to the disclosed embodiment, but on the contrary, is intended to cover various modifications and equivalent arrangements included within the scope of the appended claims.

## Claims

1. A system (300) for delivering a substance into a body at a substance-delivery site, the system comprising:
an alternating current source (308);
a plurality of electrodes (302, 304, 306); and,
circuitry connected between the alternating current source (308) and the electrodes (302, 304, 306) for supplying current to the electrodes when the electrodes are in electrical contact with the body and comprising means for maintaining a unidirectional current flow at the substance-delivery site for delivering the substance into the body, and means for maintaining a bi-directional current flow through the body.

2. The system (300) according to claim 1, wherein the plurality of electrodes (302, 304, 306) comprises at least three electrodes including a treatment electrode (302), a counter electrode (304) and an auxiliary electrode (306).

3. The system (300) according to claim 1 or 2, wherein the electrodes are configured to be applied to animal skin (310) as a substance delivery site.

4. The system (300) according to claim 1 or 2, wherein the electrodes are configured to be applied to human skin (310) as a substance delivery site.

5. The system (300) according to claim 1 or 2, wherein the electrodes are configured to be applied to a mucosal membrane as a substance delivery site.

6. The system (300) according to any of claims 1 to 5, wherein the substance comprises medicament.

7. The system (300) according to any of claims 1 to 6, wherein the circuitry comprises one or two rectifiers (320, 322).

8. The system (300) according to any of claims 1 to 7, wherein the circuitry comprises one of more diodes (320, 322).

9. The system (300) according to any of claims 1 to 8, wherein the alternating current source has a frequency of about 100 kilohertz.

10. The system (300) according to any of claims 1 to 8, wherein the alternating current source has a frequency between about 1 kilohertz and 1 megahertz.

11. The system (300) according to any of claims 2 to 10, wherein the auxiliary electrode (306) is small compared to the treatment electrode (302).

12. The system (300) according to any of claims 1 to 11, wherein the magnitude of the uni-directional current is about 1 milliamp (RMS).

13. The system (300) according to any of claims 1 to 11, wherein the magnitude of the uni-directional current is between about 10 microamps (RMS) and 1 milliamp (RMS).

14. The system (300) according to any of claims 1 to 13, wherein:
the plurality of electrodes (302, 304, 306) comprises first (302) and second (306) electrodes coupled to a first terminal of the alternating current source (308), and a third electrode (304) coupled to a second terminal of the alternating current source (308);
the circuitry comprises a first rectifying element (320) coupled between the first electrode (302) and the first terminal of the alternating current source (308), and a second rectifying element (322) coupled between the second electrode (306) and the first terminal of the alternating current source (308);
the first electrode is adapted for electrical contact with the substance-delivery site; and,
when the electrodes (302, 304, 306), including the first electrode (302) are in electrical contact with the body:
a current rectified by the first rectifying element (320) flows in a current path between the first (302) and third (304) electrodes during first cycle portions of current generated by the alternating current source (308) so as to effect delivery of the substance into the body at the substance-delivery site; and,
a current rectified by the second rectifying element (322) flows in a current path between the second (306) and third (304) electrodes during second cycle portions of the current generated by the alternating current source (308).

15. The system (300) according to claim 14, wherein the first and second rectifying elements (320, 322) comprise diodes.

## Patentansprüche

1. System (300) zum Freisetzen oder Verabreichen einer Substanz in einen Körper an einer Substanz-Verabreichungs-Stelle, wobei das System aufweist:
eine Wechselstromquelle (308);
eine Mehrzahl von Elektroden (302, 304, 306); und
eine Schaltung, welche zwischen der Wechselstromquelle (308) und den Elektroden (302, 304, 306) verbunden ist, zum Zuführen von Strom zu den Elektroden, wenn die Elektroden in elektrischem Kontakt mit dem Körper sind und eine Vorrichtung aufweist, um einen einseitig gerichteten Stromfluss bei der Substanz-Verabreichungs-Stelle aufrecht zu erhalten zur Verabreichung der Substanz in den Körper, und eine Vorrichtung zum Aufrechterhalten eines bidirektionalen Stromflusses durch den Körper.

2. System (300) nach Anspruch 1, wobei die Mehrzahl der Elektroden (302, 304, 306) mindestens drei Elektroden aufweist einschließlich einer Behandlungselektrode (302), einer Gegenelektrode (304) und einer Hilfselektrode (306).

3. System (300) nach Anspruch 1 oder 2, wobei die Elektroden so konfiguriert sind, um an einer Tierhaut (310) als Substanz-Verabreichungs-Stelle angewendet zu werden.

4. System (300) nach Anspruch 1 oder 2, wobei die Elektroden so konfiguriert sind, um an menschlicher Haut (310) als Substanz-Verabreichungs-Stelle angewendet zu werden.

5. System (300) nach Anspruch 1 oder 2, wobei die Elektroden so konfiguriert sind, um an einer Schleimhaut-Membran (310) als Substanz-Verabreichungs-Stelle angewendet zu werden.

6. System (300) nach einem der Ansprüche 1 bis 5, wobei die Substanz ein Medikament enthält.

7. System (300) nach einem der Ansprüche 1 bis 6, wobei die Schaltung einen oder zwei Gleichrichter (320, 322) aufweist.

8. System (300) nach einem der Ansprüche 1 bis 7, wobei die Schaltung eine oder mehr Dioden (320, 322) aufweist.

9. System (300) nach einem der Ansprüche 1 bis 8, wobei die Wechselstromquelle eine Frequenz von ungefähr 100 Kilohertz hat.

10. System (300) nach einem der Ansprüche 1 bis 8, wobei die Wechselstromquelle eine Frequenz zwischen ungefähr einem Kilohertz und einem Megahertz hat.

11. System (300) nach einem der Ansprüche 2 bis 10, wobei die Hilfselektrode (306) im Vergleich zu der Behandlungselektrode (302) klein ist.

12. System (300) nach einem der Ansprüche 1 bis 11, wobei die Größe des einseitig gerichteten Stroms ungefähr ein Milliampere (RMS; Effektivwert) ist.

13. System (300) nach einem der Ansprüche 1 bis 11, wobei die Größe des einseitig gerichteten Stromes zwischen ungefähr 10 Mikroampere (RMS) und einem Milliampere (RMS) ist.

14. System (300) nach einem der Ansprüche 1 bis 13, wobei:
die Mehrzahl der Elektroden (302, 304, 306) erste (302) und zweite (306) Elektroden aufweist, welche mit einem ersten Anschluss der Wechselstromquelle (308) gekoppelt sind, und eine dritte Elektrode (304), welche mit einem zweiten Anschluss der Wechselstromquelle (308) gekoppelt ist;
die Schaltung weist ein erstes Gleichrichterelement (320) auf, welches zwischen der ersten Elektrode (302) und dem ersten Anschluss der Wechselstromquelle (308) gekoppelt ist, und ein zweites Gleichrichterelement (322), welches zwischen der zweiten Elektrode (306) und dem ersten Anschluss der Wechselstromquelle (308) gekoppelt ist;
die erste Elektrode ist ausgelegt für einen elektrischen Kontakt mit der Substanz-Verabreichungs-Stelle; und wenn die Elektroden (302, 304, 306) einschließlich der ersten Elektrode (302) im elektrischen Kontakt mit dem Körper stehen:
fließt ein Strom, welcher durch das erste Gleichrichterelement (320) gleichgerichtet wurde, in einem Strompfad zwischen den ersten (302) und dritten (304) Elektroden während ersten Zyklusabschnitten des Stroms, erzeugt durch die Wechselstromquelle (308), um so eine Verabreichung der Substanz in den Körper bei der Substanz-Verabreichungs-Stelle zu bewirken; und
fließt ein Strom gleichgerichtet durch das zweite Gleichrichterelement (322) in einem Strompfad zwischen den zweiten (306) und dritten (304) Elektroden während zweiten Zyklusabschnitten des Stromes, erzeugt durch die Wechselstromquelle (308).

15. System (300) nach Anspruch 14, wobei die ersten und zweiten Gleichrichterelemente (320, 322) Dioden aufweisen.

## Revendications

1. Système (300) destiné à l'administration d'une substance dans un corps au niveau d'un site d'administration d'une substance, le système comprenant :
une source de courant alternatif (308) ;
une pluralité d'électrodes (302, 304, 306) ; et,
des circuits reliés entre la source de courant alternatif (308) et les électrodes (302, 304, 306) pour approvisionner les électrodes en courant quand les électrodes sont en contact électrique avec le corps et comprenant un moyen de maintenir un débit de courant unidirectionnel au niveau du site d'administration de la substance pour délivrer la substance dans le corps et un moyen de maintenir un débit de courant bidirectionnel à travers le corps.

2. Système (300) selon la revendication 1, dans lequel la pluralité d'électrodes (302, 304, 306) comprend au moins trois électrodes comprenant une électrode de traitement (302), une contre-électrode (304) et une électrode auxiliaire (306).

3. Système (300) selon la revendication 1 ou 2, dans lequel les électrodes sont configurées pour être appliquées à une peau animale (310) comme site d'administration d'une substance.

4. Système (300) selon la revendication 1 ou 2, dans lequel les électrodes sont configurées pour être appliquées à une peau humaine (310) comme site d'administration d'une substance.

5. Système (300) selon la revendication 1 ou 2, dans lequel les électrodes sont configurées pour être appliquées à une membrane muqueuse comme site d'administration d'une substance.

6. Système (300) selon l'une quelconque des revendications 1 à 5, dans lequel la substance comprend un médicament.

7. Système (300) selon l'une quelconque des revendications 1 à 6, dans lequel les circuits comprennent un ou deux redresseurs (320, 322).

8. Système (300) selon l'une quelconque des revendications 1 à 7, dans lequel les circuits comprennent une ou plusieurs diodes (320, 322).

9. Système (300) selon l'une quelconque des revendications 1 à 8, dans lequel la source de courant alternatif présente une fréquence d'environ 100 kilohertz.

10. Système (300) selon l'une quelconque des revendications 1 à 8, dans lequel la source de courant alternatif présente une fréquence comprise entre environ 1 kilohertz et 1 mégahertz.

11. Système (300) selon l'une quelconque des revendications 2 à 10, dans lequel l'électrode auxiliaire (306) est petite comparée à l'électrode de traitement (302).

12. Système (300) selon l'une quelconque des revendications 1 à 11, dans lequel l'intensité du courant unidirectionnel est d'environ 1 milliampère (RMS).

13. Système (300) selon l'une quelconque des revendications 1 à 11, dans lequel l'intensité du courant unidirectionnel est comprise entre environ 10 microampères (RMS) et 1 milliampère (RMS).

14. Système (300) selon l'une quelconque des revendications 1 à 13, dans lequel :
la pluralité d'électrodes (302, 304, 306) comprend des première (302) et deuxième (306) électrodes couplées à une première borne de la source de courant alternatif (308), et une troisième électrode (304) couplée à une deuxième borne de la source de courant alternatif (308) ;
les circuits comprennent un premier élément de redressement (320) couplé entre la première électrode (302) et la première borne de la source de courant alternatif (308) et un second élément de redressement (322) couplé entre la deuxième électrode (306) et
la première borne de la source de courant alternatif (308) ;
la première électrode est adaptée pour un contact électrique avec le site d'administration de substance ; et,
lorsque les électrodes (302, 304, 306), y compris la première électrode (302), sont en contact électrique avec le corps :
un courant redressé par le premier élément de redressement (320) s'écoule dans un chemin de courant entre les première (302) et troisième (304) électrodes pendant les premières parties du cycle du courant généré par la source de courant alternatif (308) afin d'effectuer l'administration de la substance dans le corps au niveau du site d'administration de substance; et,
un courant rectifié par le second élément de redressement (322) s'écoule dans un chemin de courant entre les deuxième (306) et troisième (304) électrodes pendant les deuxièmes parties du cycle du courant généré par la source de courant alternatif (308).

15. Système (300) selon la revendication 14, dans lequel les premier et second éléments de redressement (320, 322) comprennent des diodes.
